(19) Europäisches Patentamt
European Patent Office
Office européen des brevets

(11) EP 1 056 475 B1

(12) **EUROPÄISCHE PATENTSCHRIFT**

(45) Veröffentlichungstag und Bekanntmachung des Hinweises auf die Patenterteilung:
**02.05.2003 Patentblatt 2003/18**

(21) Anmeldenummer: **99904807.7**

(22) Anmeldetag: **23.01.1999**

(51) Int Cl.[7]: **A61K 47/48**, A61P 25/00

(86) Internationale Anmeldenummer:
**PCT/EP99/00439**

(87) Internationale Veröffentlichungsnummer:
**WO 99/043358 (02.09.1999 Gazette 1999/35)**

(54) **DENDRIMERE FULLERENDERIVATE, VERFAHREN ZUR HERSTELLUNG UND VERWENDUNG ALS NEUROPROTEKTIVA**

DENDRIMERIC FULLERENE DERIVATIVES, METHOD FOR PRODUCING SAME AND THEIR USE AS NEUROPROTECTORS

DERIVES DE FULLERENES DENDRIMERES, LEUR PROCEDE DE PRODUCTION ET LEUR UTILISATION COMME AGENTS NEUROPROTECTEURS

(84) Benannte Vertragsstaaten:
**AT BE CH CY DE DK ES FI FR GB GR IE IT LI LU MC NL PT SE**

(30) Priorität: **25.02.1998 DE 19807979**

(43) Veröffentlichungstag der Anmeldung:
**06.12.2000 Patentblatt 2000/49**

(73) Patentinhaber: **Siemens Axiva GmbH & Co. KG
65926 Frankfurt am Main (DE)**

(72) Erfinder: **HIRSCH, Andreas
D-91074 Herzogenaurach (DE)**

(74) Vertreter: **Berg, Peter, Dipl.-Ing. et al
European Patent Attorney,
Siemens AG,
Postfach 22 16 34
80506 München (DE)**

(56) Entgegenhaltungen:

- **BRETTREICH, MICHAEL ET AL: "A highly water -soluble dendro[60] fullerene" TETRAHEDRON LETT. (1998), 39(18), 2731-2734 CODEN: TELEAY;ISSN: 0040-4039, 30. April 1998, XP002105119**
- **DUGAN, L.L. ET AL.: "Carboxyfullerenes as neuroprotective agents." PROCEEDINGS OF THE NATIONAL ACADEMY OF SCIENCES OF USA, Bd. 94, 1997, Seite 9434 XP002105120 WASHINGTON US**
- **DATABASE MEDLINE US NATIONAL LIBRARY OF MEDICINE (NLM), BETHESDA, MD, US AN=93115740, KARFUNKEL H R ET AL: "Heterofullerenes: structure and property predictions, possible uses and synthesis proposals." XP002105123 & JOURNAL OF COMPUTER-AIDED MOLECULAR DESIGN, (1992 OCT) 6 (5) 521-35. JOURNAL CODE: JCB. ISSN: 0920-654X., Netherlands**
- **DATABASE MEDLINE US NATIONAL LIBRARY OF MEDICINE (NLM), BETHESDA, MD, US DN=98161502, CHEN H H ET AL: "Acute and subacute toxicity study of water-soluble polyalkylsulfonated C6 in rats." XP002105124 & TOXICOLOGIC PATHOLOGY, (1998 JAN-FEB) 26 (1) 143-51. JOURNAL CODE: TOY. ISSN: 0192-6233., United States**
- **NIERENGARTEN, JEAN FRANCOIS ET AL: "Macrocyclization on the fullerene core. Direct regio- and diastereoselective multi-functionalization of [60]fullerene, and synthesi of fullerene-dendrimer derivatives" HELV. CHIM. ACTA (1997), 80(7), 2238-2276 CODEN: HCACAV;ISSN: 0018-019X, XP002105121**
- **CARDULLO, FRANCESCA ET AL: "Stable Langmuir and Langmuir-Blodgett Films of Fullerene -Glycodendron Conjugates" LANGMUIR (1998), 14(8), 1955-1959 CODEN: LANGD5;ISSN: 0743-7463, XP002105122**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- DATABASE CHEMABS CHEMICAL ABSTRACTS SERVICE, COLUMBUS, OHIO, US DN=128:315602, DAI, LIMING ET AL: "Doping of conducting polymers by sulfonated fullerene derivatives and dendrimers" XP002105125 & J. PHYS. CHEM. B (1998), 102(21), 4049-4053 CODEN: JPCBFK;ISSN: 1089-5647,

## Beschreibung

Die Erfindung betrifft dendrimere Fullerenderivate, das heißt Fullerene, die mit einem oder mehreren Dendronen substituiert sind. Fullerene sind Kohlenstoffverbindungen der allgemeinen Formel $C_{2(10+m)}$, wobei m eine natürliche Zahl ist. Sie enthalten zwölf Fünf- sowie beliebig viele d.h. m, mindestens aber zwei, Sechsringe aus Kohlenstoffatomen.

Die nach außen gewölbte Oberfläche von Fullerenen und die dadurch hervorgerufene Ausrichtung der $\pi$-Elektronen bedingen eine starke Reaktivität gegenüber freien Radikalen /1/ (Die Literatur-Fundstellen sind am Schluß der Beschreibung aufgeführt). So ist für das $C_{60}$-Buckminster-Fulleren bekannt, daß es sehr leicht Radikale addiert. Die Aufnahme von bis zu 34 Methylradikalen ist beschrieben und es wurde dafür die Bezeichnung "Radikalschwamm" vorgeschlagen /1/.

Es ist bekannt, daß Hydroxyl- und Hydroperoxidradikale in biologischen Systemen bevorzugt mehrfach ungesättigte Fettsäuren angreifen. Dieser Angriff bewirkt eine Vernetzung und Polymerisation der Fettsäurestrukturen. Damit bewirken freie Radikale auch eine Schädigung von Biomembranen, insbesondere der Membranen von Nervenzellen, die sich durch einen besonders hohen Anteil an ungesättigten Fettsäuren in ihren Lipiden auszeichnen. Als Folge der Veränderung der Lipidstruktur solcher Biomembranen kann eine Veränderung der Membrandurchlässigkeiten, eine Änderung ihrer Transportfunktionen, Wechsel ihrer Barrieremechanismen, Änderungen ihrer Rezeptoraktivitäten auftreten und es kann zum Tod der Nervenzelle führen. Überstimulation von Glutamatrezeptoren führte ebenfalls zur Bildung von freien Sauerstoffradikalen und Stickoxidradikalen /2-6/ und als Folge zur Schädigung von Nervenzellen und zum Auftreten bestimmter Krankheitsbilder /7-10/.

Radikalfänger sorgen für die Entfernung schädigender freier Radikale, wirken als Antioxidantien und unterbinden die Schädigung der Nervenzellen und den Funktionsverlust von Neuromembranen. Daher sollten gerade Fullerene mit ihren ausgezeichneten Radikalfängereigenschaften als Neuroprotektiva wirken und sich zum Schutz von biologischen Membranen gegenüber oxidativen Veränderungen einsetzen lassen. $C_{60}$-Fulleren ist jedoch nur in ganz wenigen organischen Lösungsmitteln wie Benzol und Toluol löslich und kann damit auch nicht in biologische Membranen appliziert werden. Die Addition von geeigneten funktionellen Gruppen an $C_{60}$ sollte jedoch die Wasserlöslichkeit verbessern und damit auch die Verwendung als Antioxidantien in biologischen Systemen ermöglichen. Erste positive Ergebnisse wurden mit polyhydroxyliertem $C_{60}$ /11/ erreicht, bessere Resultate ergaben dreifach substituierte synthetische Malonsäurederivate $C_{60}[C(COOH)_2]_3$, die sowohl *in vitro* als auch *in vivo* neuroprotektive Aktivitäten aufwiesen /12/. Die in /12/ beschriebene Darstellung der beiden Isomere mit $C_3$- bzw. $D_3$-Symmetrie erfolgte nach der Methode von Hirsch /13/ durch dreifache Cyclopropanierung von $C_{60}$ mit Malonsäurediethylester, Hydrolyse und anschließender chromatographischer Reinigung. Die beiden Testverbindungen zeigten starke Affinität zu freien Radikalen, wie durch EPR-Spektroskopie gezeigt werden konnte, und inhibierten den excitotoxischen Tod von Zellkulturen kortikaler Neuronen, die durch *N*-Methyl-D-aspartat(NMDA)-Exposition, durch $\alpha$-3-Hydroxy-5-methyl-4-isoxazolpropionsäure(AMPA)-Exposition oder durch Sauerstoff-Glucose-Entzug induziert wurden. Das $C_3$-symmetrische Derivat reduzierte außerdem apoptotischen neuralen Tod, der entweder durch Serumentzug oder Exposition von $A^{\beta}$1-42-Protein induziert wurde. In allen Fällen zeigte das $C_3$-Regioisomer größere Wirksamkeit als das $D_3$-Isomer, was sich auf die größere Polarität und damit verbesserte Fähigkeit der Lipidmembranpenetration zurückführen läßt /12/.

Die Malonsäurederivate zeigten gegenüber den polyhydroxysubstituierten Fullerenen bessere Wirksamkeit als Radikalfänger, wie es mit dem geringeren Additionsgrad und daraus folgend einer vermehrten Bereitstellung einer größeren reaktiven Oberfläche mit gleichzeitig einhergehender verbesserter Wasserlöslichkeit.

Weiter sind aus /17/ Fullerene bekannt, die mit Poly(benzylether)-Dendronen verbunden sind. Diese Fullerenderivate sind jedoch in Wasser unlöslich. In /19/werden ebenfalls dendrimere Fullerenderivate offenbart, die in Wasser unlöslich sind.

Gegenüber diesem Stand der Technik lag der Erfindung die Aufgabe zugrunde, die Wasserlöslichkeit von Fullerenen bzw. Fullerenderivaten weiter zu verbessern, ohne gleichzeitig, wie bei den bis dato bekannten Fällen, die reaktive $C_{60}$-Oberfläche zu verkleinern bzw. abzuschirmen und damit die Radikalfängereigenschaften herabzusetzen.

Diese Aufgabe wird erfindungsgemäß gelöst durch dendrimere Fullerenderivate, bei denen das Fulleren mit mindestens einem Dendron verbunden ist, dadurch gekennzeichnet, daß das oder jedes Dendron mindestens eine protische Gruppe aufweist, die Wasserlöslichkeit vermittelt.

Als protische Gruppen eignen sich die dem Fachmann bekannten hydrophilen Gruppen, vorzugsweise -OH, -COOH, $-NH_2$, $-SO_3H$,$-PO_3H$, $NR_4^+$,-NHOH, oder $-SO_2NH_2$, wobei die vier Reste R unabhängig voneinander jeweils gleich oder verschieden sein können -H, alkyl oder aryl. Besonders bevorzugt sind -OH, -COOH, $-NH_2$. Die Gruppe -COH kann ebenfalls eingesetzt werden.

Die Wasserlöslichkeit der erfindungsgemäßen Fullerenderivate kann bei 25 °C und pH 7 größer sein als 1 mg/ml, bevorzugt größer als 5 mg/ml besonders bevorzugt größer als 10 g/l und ganz besonders bevorzugt größer als 20 mg/ml. Bei 25 °C und pH 10 kann sie größer sein als 10 mg/ml, bevorzugt größer als 50 mg/ml besonders bevorzugt größer als 100 g/l und ganz besonders bevorzugt größer als 200 mg/ml.

Ein Dendron im Sinne der Erfindung ist ein Addend der $C_{60}$-Kerneinheit, der am Ende eine Verzweigung als Struktureinheit besitzt. Mit dieser kann wieder jeweils die gleiche oder eine andere Untereinheit verknüpft werden, an deren anderem Ende sich ebenso wiederum eine Verzweigung befindet. Je nach Verzweigungsgrad verzwei-, drei- oder -vielfacht sich damit die Zahl der nächsten Verzweigungen und damit auch die Anzahl der funktionellen (und wasserlöslichen) Endgruppen. Jede, durch neuerliche Anknüpfung von Dendronen entstandene, radialsymmetrische "Schale" wird als nächste Generation bezeichnet. Um mehr reaktive Oberfläche für Radikale zur Verfügung zu stellen, erscheint es sinnvoll, die zur Wasserlöslichkeit notwendigen Dendronen möglichst auf Distanz zum $C_{60}$-Kern zu halten, um dessen "Bedeckung" zu verhindern. Dies gelingt durch Anbindung der Dendronen der ersten Generation über einen sog. "spacer", d.i. ein Kohlenstoffkette der Länge $C_1$ bis ca. $C_{100}$, vorzugsweise $C_2$ - $C_{10}$, die der 1. Generation als "Abstandshalter" dient.

Als Verzweigungen eignen sich vor allem drei- oder mehrbindige Elemente wie z.B. N-, C-, P-, Si, oder mehrbindige Molekülsegmente wie Aryl-, Heteroaryl. Der Grad der Verzweigung liegt vorzugsweise zwischen zwei und drei, die Zahl der Generationen kann zwischen einschließlich 1 und 10 liegen.

Bevorzugte Lösungen der erfindungsgemäßen Aufgabe ergeben sich aus den Unteransprüchen. Es können auch einzelne oder mehrere der in den Unteransprüchen offenbarten Merkmale jeweils für sich oder in Kombination Lösungen der zugrundeliegenden Aufgabe darstellen und es sind auch die einzelnen Merkmale innerhalb der Anspruchskategorien beliebig kombinierbar.

Eine Verbesserung gegenüber den weiter oben genannten Beispielen sollten Monoaddukte von Fullerenen darstellen, sofern ihre Wasserlöslichkeit ausreichend hoch ist. Je geringer der Additionsgrad der Fullerene ist, um so größer wird deren freie reaktive Oberfläche sein, die sich für die Radikalfängereigenschaften verantwortlich zeigt. Solche Monoaddukte sollten aber gegenüber Polyaddukten eine geringere Wasserlöslichkeit aufweisen, da die Zahl der hydrophilen Gruppen entsprechend abnimmt. Dieser Konflikt wird durch die erfindungsgemäßen Fullerene gelöst, da sie durch die weiter außen liegenden Verzweigungen der höheren Generationen wiederum beliebig an Wasserlöslichkeit zunehmen.

Eine bevorzugte Verbindung ist in Formel 1 (Schema 1) dargestellt. Dabei handelt es sich um dendrimerverzweigte Derivate von $C_{60}$-Fulleren. Monosubstituierte Dendrimere dieser Art bieten den Vorteil, daß sie unabhängig vom Additionsgrad durch die Wahl geeigneter funktioneller Endgruppen ihre ausgezeichnete Wasserlöslichkeit erhalten. Die Anzahl der zur Löslichkeit beitragenden Funktionen der äußersten Dendrimerschale wird durch den Grad der Verzweigung und durch die Zahl der Dendron-Generationen mit vorgegeben (vgl. oberer Abschnitt). Verzweigungselemente der einzelnen Molekülester können unter anderem vorzugsweise $\gamma$-Amino-triscarbonsäuren der in Formel **1** gezeigten Art, $\alpha$-Aminodicarbonsäuren, $\alpha$-$\bar{\omega}$-Diaminocarbonsäuren, Hydroxycarbonsäuren, Weinsäurederivate, Polyphenole, Kohlenhydratkomponenten vom Pentose- und Hexose-Typ, Glycerinderivate sowie allgemein polyfunktionelle Verbindungen darstellen. Die konsekutiven Verknüpfungen der einzelnen Dendrimeräste bzw. der einzelnen Generationen entspricht dann Säureamidbindungen, Peptidbindungen, Esterbindungen, Depsipeptid-artigen Bindungen nach Art der wechselnden Säureamid- bzw. Esterverknüpfung von Aminosäuren und Hydroxysäuren, Glyceridartigen Bindungen und Polyestern bzw. Polyethern, Polyphenylethern und Glycosiden. Erst bei Dendrimerhüllen der dritten Generation ist eine Beeinträchtigung der freien Fullerenoberfläche durch Abschirmung zu erwarten, die auf Grund leichter Penetrierbarkeit kleiner freier Radikale dennoch nicht zwingend zu einer Abnahme der Antioxidantienwirksamkeit führen muß. Durch die Wahl geeigneter Längen von zwei bis zu sechs Kohlenstoffatomen bei den Spacerketten kann die Verfügbarkeit der Fullerenoberfläche zusätzlich variiert bzw. gewährleistet werden. Die Verwendung langer Fettsäureketten in den einzelnen Ästen kann zusätzlich eine Affinität zu den Fettsäureketten der Lipide innerhalb der Membrandoppelschicht bewirken und läßt damit verbesserte Interkalation in Nervenmembranen und verbesserte Radikalfängereigenschaften erwarten.

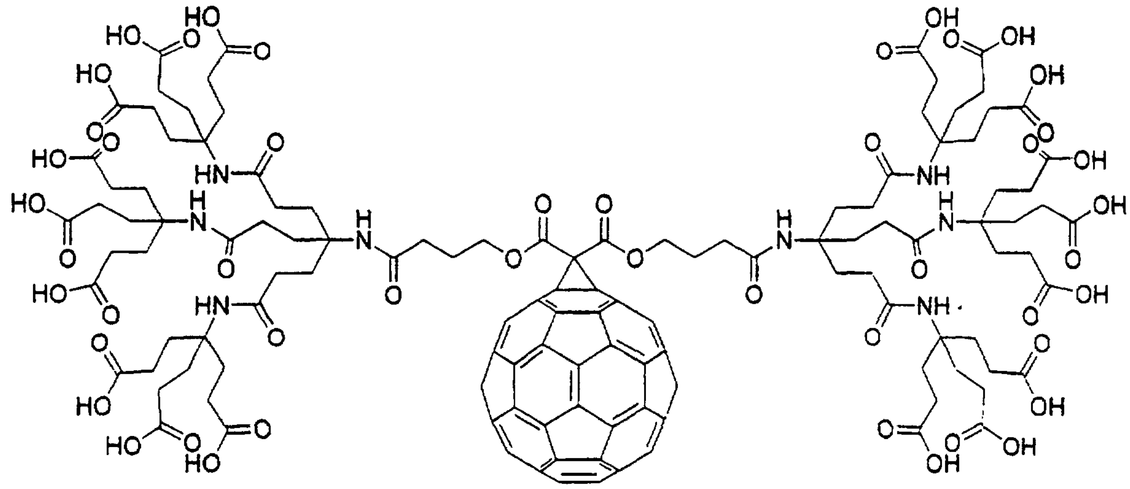

1

Schema 1

Die Darstellung der Zielverbindungen gelingt durch konvergente Synthese, d. h. der entsprechende Dendrimerast wird bis zur ersten, zweiten, dritten bzw. n-ten Generation getrennt synthetisiert und erst abschließend durch eine geeignete Verknüpfungsreaktion, wie z.B. eine Cyclopropanierung, Diels-Alder-Reaktion, [3+2]-Cycloaddition u. ä., an $C_{60}$-Fulleren gebunden. Zur Synthese des Fullerendendrimeren der ersten Generation (vgl. Herstellungsbeispiel **8**) nach Schema 2 wird die O-benzylgeschützte γ-Hydroxybuttersäure **2** mit der carboxygeschützten γ-Amino-triscarbonsäure [Behera-Amin, 4-Amino-4-(2-*t*-butyloxycarbonylethyl)-heptandisäuredi-*t*-butylester **3** (/14/, /15/, /18/)] durch Umsetzung mit Kondensationsmitteln und Aktivatoren wie z.B. Dicyclohexylcarbodiimid [DCD], 1-Hydroxybenzotriazol [1-OH-BT], jedoch auch anderen Carbodiimiden, N-Hydroxyverbindungen, u. ä. kondensiert. Das daraus resultierende Säureamid [4-(4-Benzyloxy-butyrylamino)-4-(2-*t*-butyloxycarbonylethyl)-heptandisäuredi-*t*-butylester **4**] kann durch Hydrogenolyse oder spezifischer, schonender Hydrolyse zum freien Hydroxysäureamid [4-(4-Hydroxybutyrylamino)-4-(2-*t*-butyloxycarbonylethyl)-heptandisäuredi-*t*-butylester **5**] entschützt werden, ohne daß dabei die Schutzgruppen der Carboxylfunktionen in Mitleidenschaft gezogen werden. Die folgende Umsetzung von **5** mit Malonsäuredichlorid und Pyridin oder anderen Stickstoffbasen führt zum Dendron **6** der ersten Generation [Nomenklatur für Kaskadenpolymere: 6-Cascade:Methan[2]:(2-aza-7-oxa-3,8-dioxooctylidyne):propansäure-*tert*-butylester], das einen Malonsäuredialkylester darstellt mit amidartiger Dendronverknüpfung und einer $C_4$-langen Spacer-Kette zwischen der Verzweigungseinheit und dem Malonatrest. Durch Wahl entsprechend anderer benzylgeschützter ω-Hydroxycarbonsäuren **2** wie z.B. 3-Hydroxypropionsäure, 5-Hydroxyvaleriansäure oder 6-Hydroxyhexansäure kann die Spacerkette auch beliebig verkürzt oder verlängert werden. Nach einem von uns beschriebenen Verfahren /16/ läßt sich $C_{60}$ nun in Gegenwart von $CBr_4$ und DBU/Methylenchlorid mit Synthon **6** cyclopropanieren. Die *tert*-Butyl-Schutzgruppen des entstandenen

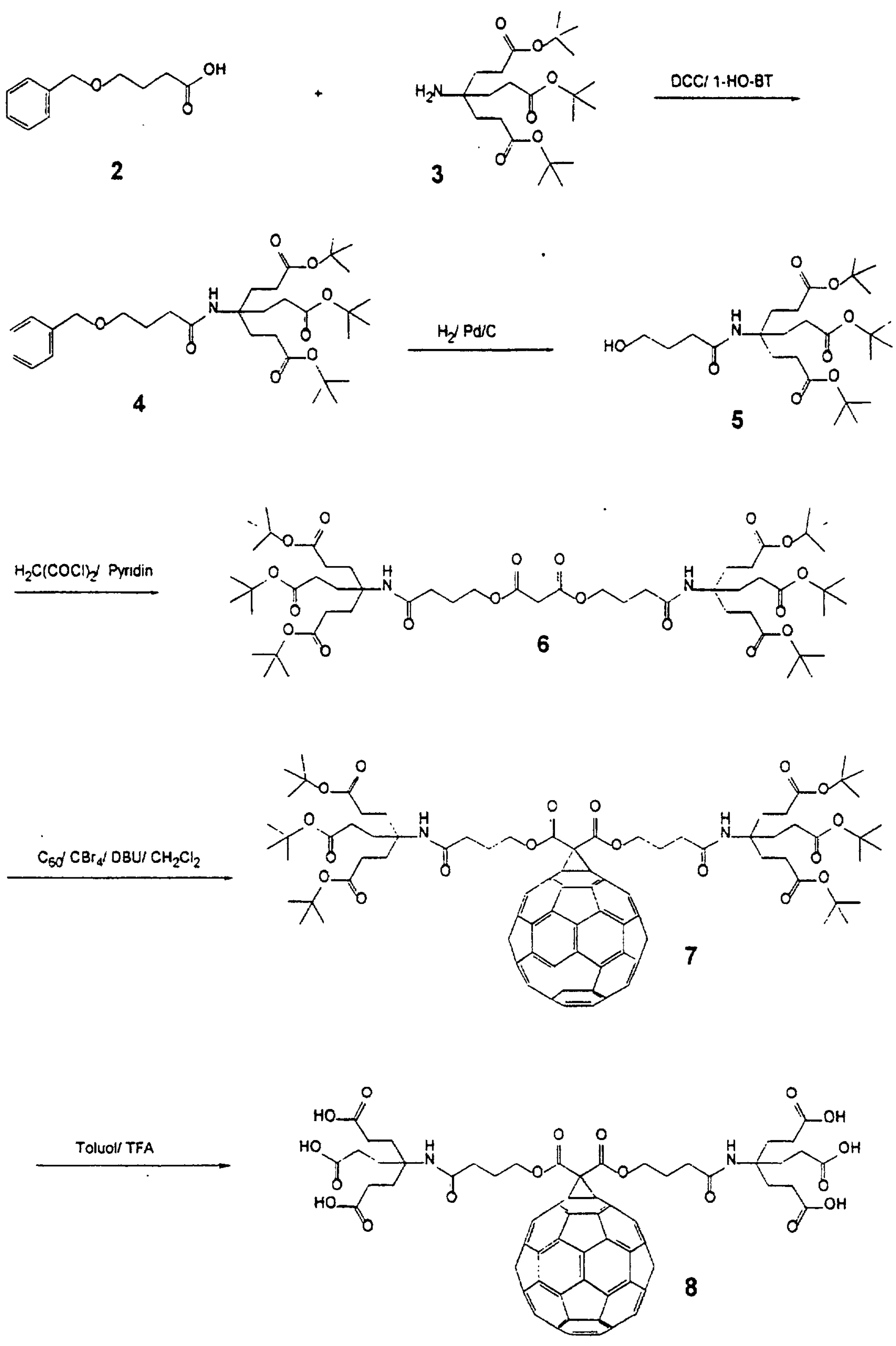


Schema 2

Dendrimerfullerens [6-Cascade:Methano-$C_{60}$-fulleren[2]:(2-aza-7-oxa-3,8-dioxooctylidyne):propansäure-*tert*-butylester **7**] lassen sich durch Hydrolyse, vorzugsweise mit Ameisensäure leicht abspalten, so daß das wasserlösliche und als Neuroprotektivum einsetzbare polycarboxyfunktionalisierte Dendrimerfulleren **8** entsteht. [6-Cascade:

Methano-$C_{60}$-fulleren[2]:(2-aza-7-oxa-3,8-dioxooctylidyne):propansäure.

Analog wie die aufgezeigte Synthese des Derivats erster Generation **8** in Schema 2 wird das Dendrimerfulleren **1** zweiter Generation [18-Cascade:Methano-$C_{60}$fulleren[2]:(2-aza-7-oxa-3,8-dioxooctylidyne):(2-aza-3-oxopentylidyne):propansäure] (vgl. Herstellungsbeispiel **1**) dargestellt (Schema 3). Wie im ersten Fall wird die *tert*butylgeschützte γ-Amino-triscarbonsäure **3** mit der entsprechenden Nitrotriscarbonsäure **9,** deren *tert*-Butylester die Synthesevorstufe von **3** darstellt (15, 16), mit DCC in Anwesenheit von 1 HO-BT und anschließender Reduktion mit Wasserstoff zum Dendron **10** zweiter Generation [9-Cascade:Aminomethan[3]:(2-aza-3-oxopentylidyne):propansäure-*tert*-butylester] umgesetzt. Die analogen Reaktionen wie zur Darstellung von **8** führen zur Verknüpfung mit dem Spacer **2**, Entstehung des entsprechenden Malonsäurediesters und Cyclopropanierung zu einem carboxylgeschützten Dendrimerfullerenderivat. Die analoge Abspaltung der Schutzgruppen führt zu dem Dendrimerfulleren **1** der zweiten Generation. Letzteres ist bereits sehr gut in Wasser (pH > 7), MeOH und anderen polaren Lösungsmitteln löslich. In Wasser wurde für die genannte Verbindung bei 25 °C und pH 7 eine Löslichkeit von ca. 34 mg/ml gemessen. Bei pH 10 betrug die Löslichkeit ca. 250 mg/ml.

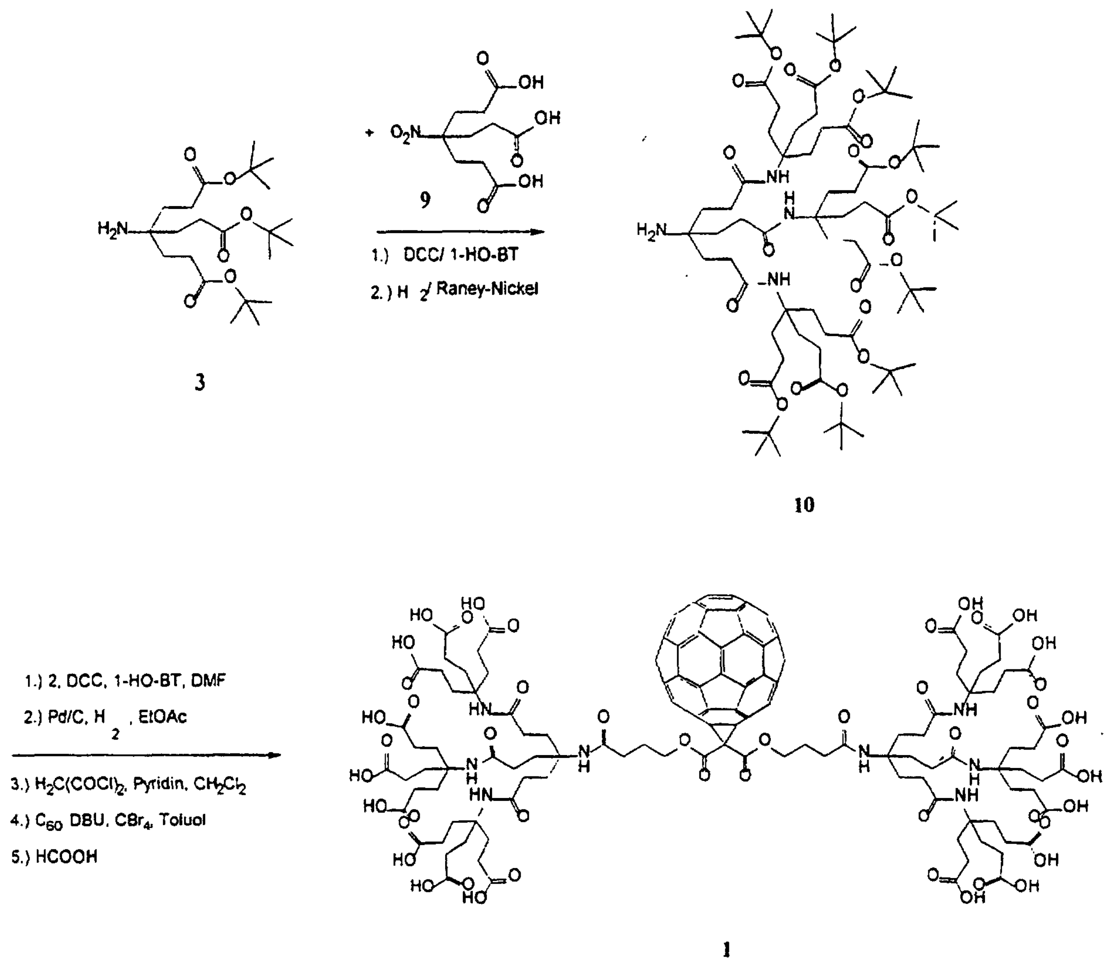


3

9

10

1

Schema 3

Eine dritte Kondensationsreaktion zwischen dem Aminoderivat **10** und der Nitrotriscarbonsäure **9** führt zu

einem Dendron **11** der dritten Generation [27-Cascade:Aminomethan[3]:(2-aza-3-oxopentylidyne)$^2$:propansäure-*tert*-butylester], das sich wiederum durch Spacer-Verlängerung in das Cyclopropanierungsreagens **12** der dritten Generation umsetzen läßt. Addition an Fulleren $C_{60}$ unter den bereits beschriebenen Bedingungen liefert ein Dendrimer **13** der dritten Generation mit einem $C_{60}$ als Core-Einheit. Entschützung der *tert*-Butylgruppen liefert wiederum ein wasserlösliches Dendrimerfulleren [54-Cascade:Methanofulleren[2]:(2-aza-7-oxa-3,8-dioxooctylidyne):(2-aza-3-oxopentylidyne)$^2$:propansäure **14**] (Schema 4) der dritten Generation, das sich durch Radikalfängereigenschaften auszeichnen sollte und membranschützende und neuroprotektive Eigenschaften besitzen sollte.

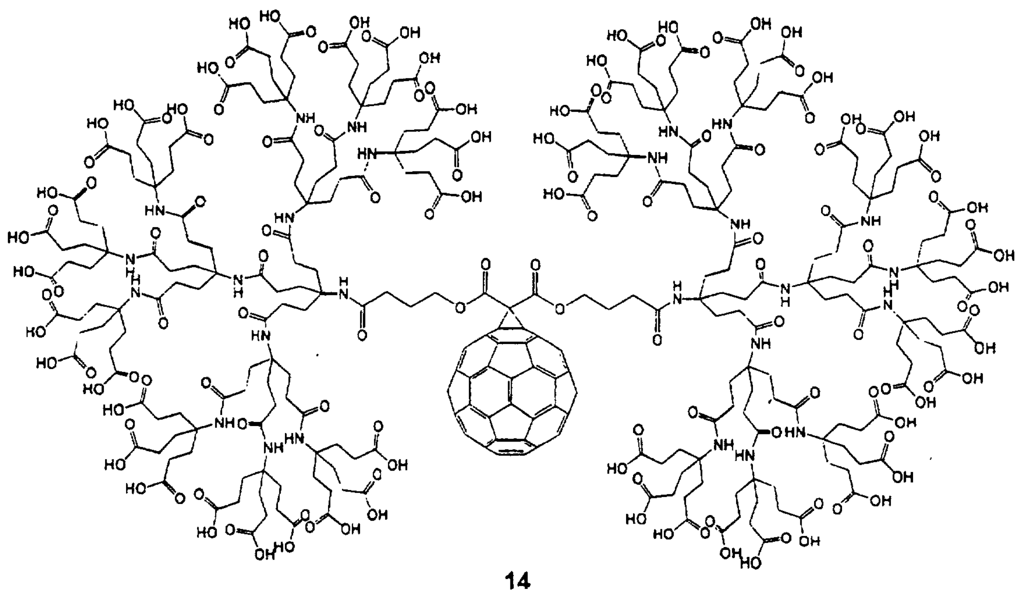

**14**

Scheme 4

Herstellungsbeispiele:

Die Numerierung der Substanzen ergibt sich aus Schema 2, wobei der Index ***a*** die entsprechende Verbindung der zweiten Generation bezeichnet.

9-Cascade:[N-(4-benzyloxybutyryl)-aminomethan][3]:(2-aza-3-oxopentylidyne): propansäure-*tert* -butylester **4 a**

Man löst 4,58 g Aminocarbonsäureester **10** [9-Cascade:aminomethan[3]:(2-aza-3-oxopentylidyne):propansäure-*tert*-butylester **10**], 618 mg 4-Benzyloxybuttersäure **2**, 656 mg Dicyclohexylcarbodiimid [DCC] und 430 mg 1-Hydroxybenzotriazol [1-HO-BT] in 50 ml DMF und läßt die Lösung 24 h bei Raumtemperatur. rühren. Nach Entfernen des Lösungsmittels i. Vak. (im Vakuum) nimmt man den Rückstand in Ethylacetat auf und wäscht mit 10 %iger kalter Salzsäure, Wasser, 10 proz.-iger $NaHCO_3$-Lösung und konz. NaCl-Lösung. Nach Trocknen über $MgSO_4$ entfernt man das Lösungsmittel i. Vak. und reinigt das Rohprodukt mittels Säulenchromatographie ($SiO_2$, Hexan/Ethylacetat 1:1). Man erhält *3,07 g* eines weißen Pulvers (60 %) **4 a.**

9-Cascade:[N-(4-hydroxybutyryl)-aminomethan][3]:(2-aza-3-oxopentylidyne): propansäure-*tert* -butylester **5 a**

Man löst 2,97 g **4 a** in 100 ml Ethanol und versetzt die Lösung mit 100 mg Paladium/Kohlenstoff (10 %). Es wird bei Raumtemperatur und Normaldruck hydriert, nach Abfiltrieren des Katalysators wird das Reaktionsgemisch i. Vak. eingeengt und man erhält 2,66 g (95 %) weißes Pulver **5 a.**

18-Cascade:Methan[2]:(2-aza-7-oxa-3,8-dioxooctylidyne):(2-aza-3-oxopentylidyne): propansäure-*tert*-butylester **6 a**

Unter Stickstoff als Schutzgas werden 1,6 g **5 a** und 83 mg wasserfreies Pyridin in 50 ml Methylenchlorid gelöst, gerührt und auf 0 °C gekühlt. 73 mg Malonsäuredichlorid werden langsam zugegeben, weitere 2 h bei 0 °C

gerührt und 12 h bei Raumtemperatur. Anschließend versetzt man mit 50 ml Methylenchlorid und wäscht mit Wasser. Nach Trocknen über $MgSO_4$ und Reinigung mittels Säulenchromatographie ($SiO_2$, Cyclohexan/Ethylacetat) erhält man 425 mg (26 %) Produkt **6 a.**

18-Cascade:methano-$C_{60}$-fulleren[2]:(2-aza-7-oxa-3,8-dioxooctylidyne):(2-aza-3-oxopentylidyne): propansäure-*tert* -butylester **7 a**

In einer Stickstoffatmosphäre werden 425 mg **6 a,** 98 mg $C_{60}$, 45 mg $CBr_4$ und 23 mg DBU in 100 ml absolutem Toluol gelöst. Man läßt 22 h bei Raumtemperatur rühren, trennt anschließend das Lösungsmittel i. Vak. ab und reinigt den Rückstand mittels Säulenchromatographie. Dazu wird zunächst nichtumgesetztes $C_{60}$ mit Toluol eluiert und danach ein Produktgemisch mit Toluol/Ethylacetat (1:1). Dieses wird mit HPLC weiter aufgereinigt. Man erhält 110 mg (21 %) braunes Pulver **7 a.**

18-Cascade:methano-$C_{60}$-fulleren[2]:(2-aza-7-oxa-3,8-dioxooctylidyne):(2-aza-3-oxopentylidyne): propansäure 1

100 mg **7 a** werden in 15 ml Ameisensäure gelöst und bei Raumtemperatur gerührt. Die Reaktion ist nach 20 h beendet. Nach Entfernen der Ameisensäure im Ölpumpenvakuum erhält man 70 mg (95 %) eines rotbraunen Pulvers **1**.

Spektroskopische Daten von **1**:

**1**: $^1$H-NMR (400 MHz, $D_2O$ + $K_2CO_3$, 25 °C) δ (ppm) 1.91 (m, 48 H), 2.13 (m, 52 H), 2.47 (t, 4 H), 4.78 (t, 4 H); $^{13}$C-NMR (100 MHz, $D_2O$ + $K_2CO_3$, 25 °C) δ (ppm) 23.96, 30.95, 31.18, 31.64, 31.87, 32.06, 52.76, 58.21, 58.40, 67.62, 71.53, 138.56, 141.54, 141.79, 142.37, 143.26, 143.52, 143.58, 144.11, 144.47, 145.07, 145.17, 145.22, 145.26, 145.35, 145.60, 145.71, 165.79, 171.45, 174.51, 175.41, 182.78; FT-IR (KBr) $\tilde{\nu}$ /$cm^{-1}$ 3344, 3074, 2925, 2854, 2625 (broad), 1712, 1654, 1542, 1458, 1414, 1268, 1231, 1206, 1103, 908, 814, 669, 527; UV-Vis ($H_2O$) $\lambda_{max}$ (ε) 257 (102615), 325 (30477), 425 (2077);

Literaturfundstellen:

/1/ Krusic, P. J., Wasserman, E., Keizer, P. N., Morton, J. R. und Preston, K. F. (1991), Science, 254, 1183-1185.

/2/ Garthwaite, J., Charles, S. L. und Chess-Williams, R. (1988), Nature (London) 336, 385-388.

/3/ Dawson, V. L., Dawson, T. M., London, E. D., Bredt, D. S. und Snyder, S. H. (1990), Proc. Natl. Acad. Sci. USA 88, 6368-6371.

/4/ Lafon-Cazal, M., Pietri, S., Culcasi, M. und Bockaert, J (1993), Nature (London) 1964, 535-537.

/5/ Dugan, L. L., Sensi, S. L., Canzoniero, L. M. T. , Handran, S. D., Rothman, S. M., Lin, T. T., Goldberg, M. P. und Choi, D. W. (1995) J. Neurosci. 15, 6377-6388.

/6/ Reynolds, I. J. und Hastings, G. G. (1995) J. Neurosci. 15, 3318-3327.

/7/ McGeer, E. G. und McGeer, P. L. (1976) Nature (London), 236, 517-524.

/8/ Rothman, S. M. und Olney, J. W. (1986) Ann. Neurol. 19, 105-111.

/9/ Choi, D. W. (1988) Neuron 1, 623-634.

/10/ McIntosh, T. Soares, H., Hayes, R. und Simon, R. (1988) *In Frontiers in Excitatory Amino Acid Research*, eds. Cavallo, E. A., Lehman, J. und Turski, L. (Liss., New York) pp. 653-656.

/11/ Dugan, L. L., Gabrielsen, J. K., Yu, S. P., Lin, T. S. und Choi, D. W. (1996) Neurobiol. Dis. 3 129-135.

/12/ Dugan, L. L., Turetsky, D. M., Cheng Du, Lobner, D., Wheeler, M., Almli, C. R., Shen, C. K.-F., Luh, T.-Y., Choi,

D. W. und Lin, T.-S. (1997) Proc. Natl. Acad. Sci. USA, 94, 9434-9439.

/13/ Lamparth, I. und Hirsch, A. (1994) J. Chem. Soc. Chem. Commun. 1994, 1727-1728.

/14/ Newkome, G.R., Behera, R.K., Moorefield, C.N. und Baker, G.R. (1991) J. Org. Chem. 56, 7162.

/15/ Newkome, G.R., Nayak, A., Behera, R.K., Moorefield, C.N. und Baker, G.R. (1992) J. Org. Chem. 57, 358.

/16/ Hirsch, A. und Camps, X. (1997) J. Chem. Soc.; Perkin 1 1997, 1595.

/17/ Newkome, G.R., Moorefield, C.N., Vögtle, F., Dendritic Molecules, VCH Weinheim New York Basel Cambridge Tokyo, 1996.

/18/ Camps, X., Schönberger, H. und Hirsch, A. (1997) Chemistry Eur. J. 3, 561.

/19/ J.-F. Nierengarten et. al. Helvetica Chimica Acta Vol. 80, (1997), 2238-2276.

## Patentansprüche

1. Dendrimere Fullerenderivate, bei denen das Fulleren mit mindestens einem Dendron verbunden ist, **dadurch gekennzeichnet, daß** das oder jedes Dendron mindestens eine protische Gruppe aufweist, die Wasserlöslichkeit vermittelt.

2. Fullerenderivate nach Anspruch 1, **dadurch gekennzeichnet, daß** die Zahl der protischen Gruppen, die Wasserlöslichkeit vermitteln, größer ist als 4, bevorzugt 20, besonders bevorzugt 30 und ganz besonders bevorzugt 40.

3. Fullerenderivate nach Anspruch 1 oder 2, **dadurch gekennzeichnet, daß** die oder jede protische Gruppe gleich oder verschieden ausgewählt ist aus einer Gruppe bestehend aus -OH, -COOH, $-NH_2$, $-SO_3H$,$-PO_3H$, $NR_4^+$, -NHOH,$-SO_2NH_2$ wobei die vier Reste R unabhängig voneinander jeweils gleich oder verschieden sein können -H, alkyl oder aryl.

4. Fullerenderivate nach mindestens einem der Ansprüche 1 bis 3, **dadurch gekennzeichnet, daß** die Verzweigungselemente des oder jeden Dendrons gleich oder verschieden ausgewählt sind aus einer Gruppe bestehend aus γ-Amino-triscarbonsäuren, α-Aminodicarbonsäuren, α-$\bar{\omega}$-Diaminocarbonsäuren, Hydroxycarbonsäuren, Weinsäurederivaten, Polyphenolen, Kohlenhydratkomponenten vom Pentose- oder Hexose-Typ, Glycerinderivate.

5. Fullerenderivate nach mindestens einem der Ansprüche 1 bis 4, **dadurch gekennzeichnet, daß** ihre Wasserlöslichkeit bei 25 °C und pH 7 größer ist als 1 mg/ml

6. Verfahren zur Herstellung von Verbindungen gemäß Anspruch 1 bis 5, **dadurch gekennzeichnet, daß** man in einem ersten Schritt die Dendronen herstellt und in einem zweiten Schritt über den Addenden an das Fulleren bindet.

7. Verfahren nach Anspruch 6, **dadurch gekennzeichnet, daß** man die Dendronen in einer konvergenten Synthese herstellt.

8. Mittel enthaltend Verbindungen nach einem der Ansprüche 1 bis 5 für therapeutische Zwecke.

9. Mittel enthaltend Verbindungen nach einem der Ansprüche 1 bis 5 als Neuroprotektivum.

10. Verwendung von Fullerenderivaten nach einem der Ansprüche 1 bis 5 zur Herstellung von Arzneimitteln zur Behandlung von Krankheiten, bei denen freie Radikale schädlich auf den zu behandelnden Organismus einwirken.

## Claims

1. Dendrimeric fullerene derivatives, in which the fullerene is linked to at least one dendron, **characterized in that** the or each dendron has at least one protic group which imparts water solubility.

2. Fullerene derivatives according to Claim 1, **characterized in that** the number of protic groups which impart water solubility is greater than 4, preferably 20, particularly preferably 30 and very particularly preferably 40.

3. Fullerene derivatives according to Claim 1 or 2, **characterized in that** the or each protic group is identically or differently selected from a group consisting of -OH, -COOH, $-NH_2$, $-SO_3H$, $-PO_3H$, $NR_4^+$, -NHOH, $-SO_2NH_2$, where the four radicals R independently of one another can in each case identically or differently be -H, alkyl or aryl.

4. Fullerene derivatives according to at least one of Claims 1 to 3, **characterized in that** the branching elements of the or each dendron are identically or differently selected from a group consisting of γ-aminotriscarboxylic acids, α-aminodicarboxylic acids, α-ω-diaminocarboxylic acids, hydroxycarboxylic acids, tartaric acid derivatives, polyphenols, carbohydrate components of the pentose or hexose type and glycerol derivatives.

5. Fullerene derivatives according to at least one of Claims 1 to 4, **characterized in that** its water solubility is greater than 1 mg/ml at 25°C and pH 7.

6. Process for the preparation of compounds according to Claims 1 to 5, **characterized in that** it comprises preparing the dendrons in a first step and bonding them to the fullerene via the addenda in a second step.

7. Process according to Claim 6, **characterized in that** the dendrons are prepared in a convergent synthesis.

8. Agent comprising compounds according to one of Claims 1 to 5 for therapeutic purposes.

9. Agent comprising compounds according to one of Claims 1 to 5 as neuroprotectant.

10. Use of fullerene derivatives according to one of Claims 1 to 5 for the production of pharmaceuticals for the treatment of diseases in which free radicals have a harmful effect on the organism to be treated.

## Revendications

1. Dérivés de fullerènes dendrimères, dans lesquels le fullerène est relié à au moins un dendrite, **caractérisés en ce que** le dendrite ou chaque dendrite a au moins un groupe protique qui donne de la solubilité dans l'eau.

2. Dérivés de fullerènes suivant la revendication 1, **caractérisés en ce que** le nombre des groupes protiques qui donne de la solubilité dans l'eau est supérieur à 4, de préférence est supérieur à 20, notamment de préférence à 30 et tout particulièrement de préférence à 40.

3. Dérivés de fullerènes suivant la revendication 1 ou 2, **caractérisés en ce que** le groupe ou chaque groupe protique est choisi en étant identique ou différent dans un groupe constitué de -OH, -COOH, $-NH_2$, $-SO_3H$, $-PO_3H$, $NR_4^+$, -NHOH, $-SO_2NH_2$ dans lesquels les quatre radicaux R qui peuvent être indépendamment les uns des autres respectivement identiques ou différents sont -H, alcoyle ou aryle.

4. Dérivés de fullerènes suivant au moins l'une des revendications 1 à 3, **caractérisés en ce que** les éléments de ramification du dendrite ou de chaque dendrite sont choisis en étant identiques ou différents dans le groupe constitué des acides γ-amino-triscarboxyliques, des acides α-aminodicarboxyliques, des acides α-$\bar{\omega}$-diaminodicarboxyliques, des acides hydroxycarboxyliques, des dérivés de l'acide tartrique, des polyphénols, des hydrates de carbone de type pentose ou de type hexose, des dérivés de la glycérine.

5. Dérivés de fullerènes suivant au moins l'une des revendications 1 à 4, **caractérisés en ce que** leur solubilité dans l'eau à 25°C et à pH 7 est supérieure à 1 mg/ml.

6. Procédé de préparation de composés suivant la revendication 1 à 5, **caractérisé en ce que** l'on prépare dans un premier stade les dendrites et dans un deuxième stade on les relie au fullerène par les produits d'addition.

7. Procédé suivant la revendication 6, **caractérisé en ce que** l'on prépare les dendrites dans une synthèse convergente.

8. Agent comprenant des composés suivant l'une des revendications 1 à 5 à des fins thérapeutiques.

9. Agent comprenant des composés suivant l'une des revendications 1 à 5 comme neuroprotecteurs.

10. Utilisation de dérivés de fullerènes suivant l'une des revendications 1 à 5 pour la préparation de médicaments de traitement de maladies dans lesquelles des radicaux libres agissent de manière nocive sur l'organisme à traiter.